# EUROPÄISCHE PATENTSCHRIFT

(11) **EP 0 676 391 B1**
(45) Veröffentlichungstag und Bekanntmachung des Hinweises auf die Patenterteilung: **14.10.1998**
(21) Anmeldenummer: 95104501.2
(22) Anmeldetag: 27.03.1995
(51) Int. Cl.: C07C 263/10

(54) **Verfahren zur Herstellung von Isocyanaten oder Isocyanatgemischen**
Process for the preparation of isocyanates or isocyanate mixtures
Procédé de préparation d'isocyanates ou de mélanges d'isocyanates

(30) Priorität: 07.04.1994 DE 4411911
(43) Veröffentlichungstag der Anmeldung: 11.10.1995
(73) Patentinhaber: BAYER AG, 51368 Leverkusen (DE)
(72) Erfinder: Buysch, Hans-Josef, Dr., D-47809 Krefeld (DE); Gallus, Manfred, Dr., D-47800 Krefeld (DE); Gebauer, Herbert, Dr., D-47829 Krefeld (DE); Immel, Otto, Dr., D-47803 Krefeld (DE); Mendoza-Frohn, Christine, Dr., D-40699 Erkrath (DE); Langer, Reinhard, Dr., D-47800 Krefeld (DE)

(56) Entgegenhaltungen:
- EP-A- 0 012 153
- EP-A- 0 446 781
- EP-A- 0 561 225
- GB-A- 2 207 671

## Beschreibung

Die Erfindung betrifft ein Verfahren zur Herstellung von Isocyanaten oder Isocyanatgemischen, die keine nennenswerten farbgebenden Komponenten aufweisen.

Die Beeinflussung der Farbe eines Polyurethanhartschaumes ist Grund vieler Versuche und Arbeiten. Erwünscht sind möglichst hellgelbe bis weiße Farbtöne. Neben dem besseren visuellen Eindruck lassen helle Schäume auf Rohstoffe mit hohem Reinheitsgrad schließen. Bei dunkelgelbem oder graustichigem Ausgangsmaterial bilden sich an der Hartschaumoberfläche unerwünschte Schlieren, die beim Aufsteigen während des Schäumvorganges gebildet werden. Die genaue Zusammensetzung der die Polyurethanschaumstoffarben verursachenden Farbstoffe oder farbgebenden Komponenten im Polymer ist im einzelnen noch nicht nachgewiesen worden.

Wie die Literatur zeigt, ist die Beseitigung der Farbprobleme Bestandteil der Isocyanatherstellung. Eine Vielzahl von Verfahren zur Verbesserung der Polyurethanschaumfarben wird beschrieben.

So wird in GB-A 2 207 671 die Verwendung von getrockneten Magnesiumsilikaten zur Entfernung der Spuren an "farbbildenden Verunreinigungen" im Diisocyanatodiphenylmethan (MDI) bei ca. 190°C beansprucht.

In US 4 465 639 wird das gebildete "dark colored material", welches bei der Abtrennung des Lösungsmittels bei der Diisocyanatodiphenylmethan-Herstellung entsteht, durch Zugabe von Wasser in geringen Mengen reduziert.

In US 4 876 380 wird ein Extraktionsverfahren beschrieben, bei dem Polyisocyanate mittels Methylendichlorid und Pentan in eine gereinigte Fraktion mit verbesserter Farbe und in eine Fraktion, in der Farbe keine kritische Größe ist, getrennt werden. Die Reinigung von Polyisocyanaten durch Extraktion wird auch in EP-A 0 133 538 beschrieben.

In EP-A 0 446 781 wird ein Verfahren zur Verbesserung des Polyurethanschaums beschrieben, welches sich dadurch auszeichnet, daß Diaminodiphenylmethan (MDA) einer katalytischen Hydrierung in Gegenwart von Hydrierkatalysatoren und einem bestimmten Gehalt an Wasser ausgesetzt wird, wobei als Hydrierkatalysatoren sämtliche für die katalytische Hydrierung bekannten Katalysatoren in Betracht kommen. Dieses Verfahren birgt jedoch den Nachteil in sich, daß bei der Hydrierung Nebenprodukte entstehen, die im MDA verbleiben und bei ungünstigen Phosgenierbedingungen trotzdem zu farbgebenden Verunreinigungen im MDI führen. Außerdem ist allgemein bekannt, daß durch die Phosgenierungsreaktion die Entstehung von stark gefärbten Verbindungen im ppm-Bereich nicht auszuschließen ist.

Die EP-A 561 225 zeigt, daß durch eine einfache Wasserstoffbehandlung in Gegenwart von Katalysatoren bei 3-150bar und 100 bis 180°C eine deutliche Verbesserung der Farbe der Isocyanate erreicht wird, ohne die anderen relevanten Eigenschaften der Isocyanate zu beeinträchtigen. Die Aktivität und damit die Belastbarkeit der verwendeten Katalysatoren und die erzielte Farbverbesserung lassen jedoch zu wünschen übrig.

Aufgabe der vorliegenden Erfindung war es, ein Verfahren zur Verfügung zu stellen, das es gestattet, Isocyanate oder Isocyanatgemische ohne farbgebende Stoffe in einfacher Weise und hohen Ausbeuten herzustellen.

Diese Aufgabe konnte durch das erfindungsgemäße Verfahren gelöst werden.

Gegenstand der Erfindung ist ein Verfahren zur Herstellung von Isocyanaten oder Isocyanatgemischen, die keine bzw. kaum farbgebende Stoffe enthalten, durch Phosgenierung der entsprechenden Amine oder Amingemische und anschließende Behandlung der erhaltenen Isocyanate oder Isocyanatgemische mit Wasserstoff in Gegenwart von Trägerkatalysatoren, welches dadurch gekennzeichnet ist, daß als Trägerkatalysatoren Schalenkatalysatoren eingesetzt werden, die in der kürzesten Ausdehnung 0,3 bis 3mm groß sind, BET-Oberflächen von 0,5 bis 150 m²/g aufweisen und alkalifrei sind.

Überraschenderweise wurde gefunden, daß mit dem erfindungsgemäßen Verfahren nicht nur gegenüber dem Stand der Technik hellere Isocyanate oder Isocyanatgemische hergestellt werden können, sondern daß auch höhere Durchsätze erzielt werden können und die Aktivität der Trägerkatalysatoren erhöht ist.

Geeignete Trägermaterialien sind Aktivkohle, Aluminiumoxide, Spinelle, Mischoxide, Siliciumdioxid, Titandioxid und Zirkondioxid. Spinelle und Mischoxide können z.B. Me^{II}Me^{III}₂O₄ und Me^{I}₂Me^{III}₂O₄ sein, worin Me^{I} = Li, Na, K, Me^{II} = Mn, Zn, Co, Fe, Mg, Ca, Ba und Me^{III} = Al, Cr und Fe sind.

Die Träger können verschieden geformt sein. Sie können beispielsweise als Kugeln (⌀ <3 mm), Hohlextrudate in Form von Ringen oder Raschigringen (Dicke der Ringwand <3 mm), Stäbchen (⌀ <3 mm), als sternförmige Extrudate (Dicke der Strahlen <3 mm), radförmige Extrudate (Dicke der Speichen und Reifen in der kürzesten Ausdehnung <3 mm) oder auch als unregelmäßig geformtes Bruch-oder Granulatmaterial (⌀ <3 mm), erhalten durch Aussieben mit einem Sieb der Maschenweite <3 mm, vorliegen.

Als Katalysatormetalle werden die für Hydrierungen an sich üblichen verwendet. Bevorzugt sind Platin, Palladium, Ruthenium, Iridium, Rhodium, Chrom, Mangan, Kobalt, Nickel, Silber, Gold oder Gemische davon in Mengen von 0,1 bis 5 Gew.-%, bezogen auf das Gesamtgewicht der Trägermaterialien. Besonders bevorzugt sind Platin, Palladium, Ruthenium, Rhodium und Gold. Ganz besonders bevorzugt ist eine Mischung aus Platin, Ruthenium und Gold.

Die Katalysatormetalle liegen bevorzugt am äußeren Rand der Träger vor, also beispielsweise in Form von Kugelschalen auf kugelförmigen Trägern.

Verfahren zur Herstellung von Schalenkatalysatoren sind bekannt und beispielsweise in DE-A 2 849 002, DE-A 2 848 978, DE-A 2 045 882 beschrieben. Es ist besonders darauf zu achten, daß die erfindungsgemäßen Schalenkatalysatoren frei von Alkalien sind. Die Präparation der Schalenkatalysatoren sollte daher ohne Verwendung von Alkalien durchgeführt werden, oder falls die Anwesenheit von Alkalien bei der Katalysatorherstellung unbedingt erforderlich ist, sind die Alkalien nach der Herstellung z.B. durch gründliches Waschen oder Behandlung mit Säuren zu entfernen. Als Säuren können z.B. Mineralsäuren wie HCl, H₂SO₄. H₂SO₃, Carbonsäuren wie Ameisensäure, Essigsäure, Oxalsäure oder auch Kohlensäure bzw. Kohlendioxid in Wasser verwendet werden.

Die BET-Oberfläche der Trägerkatalysatoren beträgt 0,5 bis 150 m²/g, bevorzugt 1 bis 100 m²/g, besonders bevorzugt 2 bis 80 m²/g.

Bevorzugt werden aromatische Isocyanate, insbesondere Isocyanate der Diphenylmethanreihe oder Toluylendiisocyanate bzw. deren Gemische nach dem erfindungsgemäßen Verfahren hergestellt.

Die Isocyanate werden vorzugsweise ohne Lösungsmittel eingesetzt. Unter bestimmten Voraussetzungen, wie hoher Viskosität der Isocyanate kann die Mitverwendung von Lösungsmitteln von Vorteil sein. Geeignete Lösungsmittel sind inerte Kohlenwasserstoffe und Chlorkohlenwasserstoffe, wie beispielsweise Toluol, Xylol, Ethylbenzol, Cumol, Chlorbenzol und Dichlorbenzole.

Das Verfahren kann diskontinuierlich durchgeführt werden, bevorzugt ist jedoch das kontinuierliche Verfahren z.B. in einer Rieselphase oder in einem vollständig gefluteten Katalysatorbett, wobei im letzten Fall eine Reaktorfüllung von Trägerkatalysatoren vollständig mit zu behandelnden, flüssigen Isocyanaten bedeckt ist. Dabei wird bevorzugt kein Wasserstoff durch die Isocyanat-Katalysatorfüllung geleitet, sondern die flüssigen Isocyanate werden vorher unter den Bedingungen im Reaktor mit Wasserstoff beladen, und dann das so vorbereitete Gemisch über den Katalysator geleitet. Dies kann im sinkenden wie im steigenden Flüssigkeitsstrom erfolgen.

Die Beladung mit Wasserstoff kann auf folgenden Wegen geschehen:

Das Isocyanat wird in einem Behälter bei einer Temperatur in der Nähe, bevorzugt unterhalb der der Katalysatorschüttung mit Wasserstoff begast. Die Begasung erfolgt bei einem Druck unmittelbar in der Nähe des Druckes in der Katalysatorschüttung. Danach wird die mit Wasserstoff beladene Flüssigkeit auf die Temperatur des Katalysatorbettes gebracht. Wenn hierzu aufgewärmt wird, kann ein Teil des gelösten Wasserstoffs wieder in die Gasphase entweichen. Die Aufwärmung des Wasserstoff-beladenen Isocyanats erfolgt bevorzugt so, daß gasförmiger Wasserstoff durch die Schwerkraft wieder in den Begasungsbehälter zurückgeführt wird. Der Begasungsbehälter kann ein Autoklav mit einer Begasungsvorrichtung, z.B. einem Begasungsrührer oder einer statischen Vorrichtung zur Verteilung des eingeleiteten Wasserstoffs, wie Formkörper aus porösem Sintermaterial oder perforierte Formkörper sein.

Es können auch statische Mischer in Rohren Verwendung finden, in denen Isocyanat und Wasserstoff in Kontakt gebracht, bevorzugt im Gegenstrom aneinander vorbeigeführt werden.
Statische Mischer, die hierfür geeignet sind, Formkörper zur Begasung von Flüssigkeiten und Begasungsrührer gehören zum Stand der Technik.

Bevorzugt wird in der Rieselphase gearbeitet, wobei die flüssige Phase von oben nach unten durch das Katalysatorbett rieselt und einen Flüssigkeitsfilm um die Katalysatorkörner bildet und wobei das Volumen zwischen den einzelnen Körnern Wasserstoff enthält. Die Gasphase wird von unten nach oben vorzugsweise aber von oben nach unten, also im Gleichstrom mit der Flüssigphase durch das Katalysatorbett geleitet.

Die erfindungsgemäße Behandlung mit Wasserstoff in Gegenwart von Trägerkatalysatoren wird bevorzugt bei Temperaturen von 100 bis 180°C, besonders bevorzugt von 120 bis 170°C, bei Drucken von 20 bis 200 bar, besonders bevorzugt von 50 bis 150 bar und Belastungen von 0,2 bis 10 g Isocyanat/ml Katalysator und Stunde, besonders bevorzugt von 0,5 bis 8,0 g/ml.h, ganz besonders bevorzugt von 1,0 bis 6,0 g/ml.h durchgeführt.

Die nach dem erfindungsgemäßen Verfahren hergestellten Isocyanate oder deren Gemische werden zur Herstellung von hellen Polyurethanschaumstoffen verwendet.

Die Erfindung soll anhand der nachfolgenden Beispiele näher erläutert werden.

### Beispiele

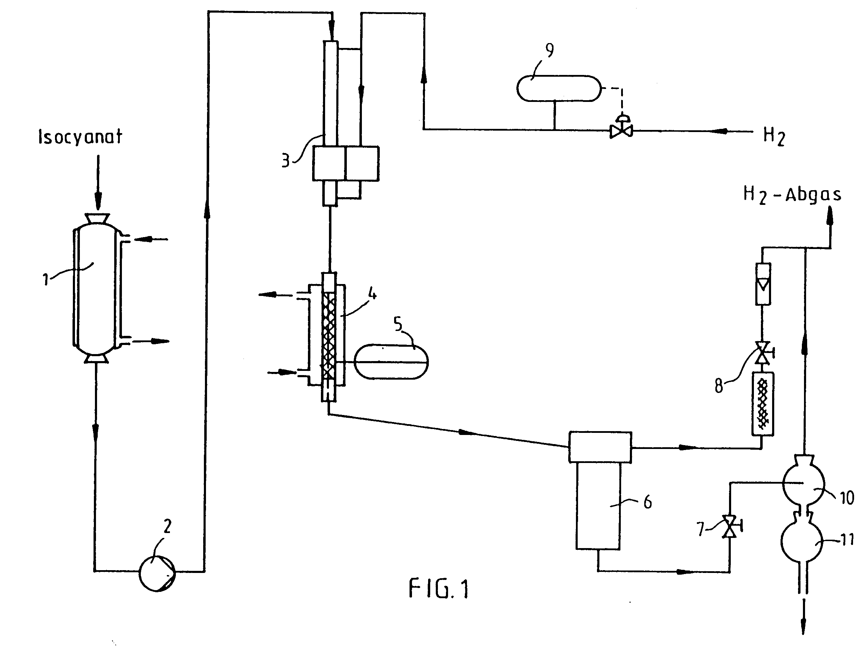

Eine kontinuierlich betriebene Wasserstoffbehandlung kann z.B. in der in Fig. 1 dargestellten Apparatur durchgeführt werden. Isocyanat wird aus dem Vorratsbehälter 1 über die Druckpumpe 2 in die Dosiervorrichtung 3 gepumpt. Der Wasserstoff wird über das Drucksteuerventil 9 von einem Vortatsbehälter auf den gewünschten Druck reduziert. In der Druckvorlage 3a wird das Isocyanat mit Wasserstoff gesättigt und fließt dann durch den Reaktionsbehälter 4 (Rieselphase), welcher mit 60 ml Katalysator gefüllt ist und bis 200°C über eine Regeleinrichtung 5 geheizt werden kann. Das mit Wasserstoff behandelte Isocyanat wird im Abscheider 6 abgetrennt und über Behälter 10 und 11 ausgeschleust. Eine kleine Menge Restwasserstoff wird über ein Ventil 8 entspannt und als Abgas gegebenenfalls zu Heizzwecken verwendet.

In das Reaktionsrohr gemäß Fig. 1 wurden für die nachfolgenden Versuche jeweils 60 ml Katalysator eingefüllt und dann das flüssige Isocyanat aus der Vorlage 3a mit Wasserstoff beladen; gefördert wird bei unterschiedlichen Belastungen in das Reaktionsrohr und bei 100 bar und 160°C mit dem Katalysator in Kontakt gebracht.

Als Isocyanat wurde ein Anfahrprodukt aus der Diphenylmethanreihe (MDI), das einen stark verfärbten Schaum ergibt, eingesetzt. Dieses MDI-Produkt besitzt die in Tabelle 1 angegebenen Extinktionen bei 430 und 520 nm, einem NCO-Gehalt von 31,4 % und eine Viskosität von 200 m.Pa.s. Die Zusammensetzung des MDI-Produktes ist wie folgt: 46,3 % 4,4'-MDI, 1,35 % 2,4'-MDI, 0,01 % 2,2'-MDI und ca. 52 % höhermolekulare MDI-Komponenten.

Die Beurteilung der Farbwerte des polymeren MDI und deren Änderungen nach der Wasserstoffbehandlung erfolgt durch die Messung der Extinktionswerte bei E = 520 nm und E = 430 nm, wobei eine Lösung von 2 g MDI in 100 ml Monochlorbenzol in einem Fotometer z.B. Digital Photometer LP 1W der Fa. Lange GmbH, Berlin vermessen wird. Diese Methode eignet sich zur schnellen Beurteilung von MDI.

Folgende Katalysatoren wurden eingesetzt:

### Katalysator A (Vergleich)

3 000 ml eines α-Aluminiumoxids*⁾ (Kugeln mit 4 bis 5 mm Durchmesser) mit einer BET-Oberfläche von 10 m²/g werden mit 156 g H₂PtCl₆- und 195 g RuCl₃ gelöst in 1 091 ml Wasser getränkt, getrocknet und bei 370°C in 20 h mit Wasserstoff reduziert. Man erhält einen Katalysator mit 13 g Pt/l und 13 g Ru/l.
*⁾ SPH 512 der Fa. Rhône Poulenc

### Katalysator B

Der nach obigem Verfahren hergestellte Katalysator A wurde grob zerkleinert und durch Sieben eine Granulatfraktion mit einem Korndurchmesser von 1 bis 2 mm erhalten.

### Katalysator C (Vergleich)

Präparation gemäß DE-B 2 849 002 Beispiel 1a, jeweils mit dem zur Füllung der Poren nötigen Volumen an Tränklösung ("incipient wetness"): Tränkung von α-Al₂O₃*⁾ (2,4 bis 4 mm Teilchendurchmesser und 10 m³/g BET-Oberfläche) mit 15 %iger NaOH, trocknen, dann tränken mit einer Lösung aus RuCl₃ und H₂PtCl₆ in H₂O (5 % Ru, 5 % Pt), trocknen, 20 h bei 370°C H₂-Reduktion. Der Katalysator enthält 4,8 g Pt/l und 3,7 g Ru/l.

### Katalysator D

Tonerdekugeln**⁾ (Durchmesser 1 mm, BET-Oberfläche 4 m²/g) werden mit einer Lösung aus RuCl₃ und H₂PtCl₆ ("incipient wetness") in H₂O (5 % Ru, 5 % Pt in Lösung) getränkt, getrocknet, 20 h bei 370°C mit H₂ reduziert. Der Katalysator enthält 5,1 g Pt/l und 3,6 g Ru/l.
**⁾ α-Al₂O₃ der Fa. Condea

### Katalysator E

α-Tonerde (siehe unter D) wird mit einer Lösung von H₂PtCl₆ + Na₂PdCl₄ in H₂O getränkt (siehe unter A).
Der Katalysator wird 10 min bei 40°C in starkem Luftstrom getrocknet, 3 h bei 350°C in H₂-Strom aktiviert. Der Katalysator enthält 13 g Pd/l und 13 g Pt/l.

### Katalysator F

γ-Al₂O₃***⁾ (BET-Oberfläche 80 m²/g) wird gebrochen und auf eine Kornfraktion von 1 bis 2 mm Durchmesser gesiebt; danach wird diese Fraktion mit H₂PtCl₆ und RuCl₃ in Wasser getränkt (siehe unter A).
Die Trocknung findet bei 40°C im Luftstrom 10 min statt, und dann wird 20 h bei 370°C im H₂-Strom aktiviert.
Der Katalysator enthält 13 g Pt/l und 13 g Ru/l.
***⁾ SPH 508 der Fa. Rhône Poulenc

### Katalysator G (Vergleich)

Präparation wie unter D, aber auf einem Träger wie unter C mit 4 % Ru und 4 % Pt in der Tränklösung.
Der Katalysator enthält 4,5 g Pt/l und 2,5 g Ru/l.

### Katalysator H (Vergleich)

Dieser Katalysator wird wie unter F hergestellt, jedoch auf einem Träger aus γ-Al₂O₃ ****) (BET-Oberfläche 300 m²/g). Der Katalysator enthält 13 g Pt/l und 13 g Ru/l.
****⁾ SPH 501 der Fa. Rhône Poulenc

### Katalysator I

Präparation wie unter F, jedoch auf gebrochenem γ-Al₂O₃*). Als Tränklösung dient eine Lösung aus 156 g H₂PtCl₆, 195 g RuCl₃ und 130 g AuCl₄ in 840 g Wasser. Der Katalysator enthält jeweils 13 g/l an Pt, Ru und Au.
*⁾ SPH 512 der Fa. Rhône Poulenc

**Tabelle 1**

| Beispiel | Katalysator | Belastung [g/ml.h] | Laufzeit des Versuches in [h] | Extinktion bei | | Abnahme der Extinktion in [%] bei | |
|---|---|---|---|---|---|---|---|
| | | | | 430nm | 520nm | 430nm | 520nm |
| 1 (Vergleich) | A | 0,3 | 166 | 0,318 | 0,048 | 9,1 | 44,2 |
| 2 | B | 0,3 | 42 | 0,194 | 0,025 | 44,6 | 70,9 |
| | | 1,1 | 39 | 0,186 | 0,026 | 46,9 | 69,8 |
| | | 2,6 | 40 | 0,212 | 0,026 | 39,4 | 69,8 |
| | | 3,5 | 45 | 0,230 | 0,025 | 34,3 | 70,9 |
| 3 (Vergleich) | C | 0,3 | 65 | 0,320 | 0,073 | 13,7 | 15,1 |
| 4 | D | 0,08 | 15 | 0,213 | 0,026 | 39,1 | 69,8 |
| | | 1,1 | 19 | 0,240 | 0,028 | 31,4 | 67,4 |
| | | 2,2 | 21 | 0,238 | 0,028 | 32,0 | 67,4 |
| 5 | E | 0,6 | 25 | 0,196 | 0,020 | 44,0 | 76,7 |
| | | 1,2 | 29 | 0,215 | 0,026 | 38,6 | 69,8 |
| | | 2,0 | 33 | 0,243 | 0,027 | 30,6 | 68,6 |
| | | 2,8 | 32 | 0,243 | 0,028 | 30,6 | 67,4 |
| | | 5,5 | 26 | 0,260 | 0,032 | 25,1 | 62,8 |
| 6 | F | 0,6 | 29 | 0,201 | 0,026 | 43,6 | 66,2 |
| | | 0,9 | 23 | 0,206 | 0,024 | 42,5 | 68,8 |
| | | 2,0 | 30 | 0,251 | 0,040 | 29,9 | 48,1 |
| 7 (Vergleich | G | 0,74 | 24 | 0,337 | 0,064 | 3,7 | 25,6 |
| 8 (Vergleich) | H | 0,5 | 50 | 0,251 | 0,025 | 29,9 | 67,5 |
| | | 0,9 | 30 | 0,266 | 0,029 | 25,7 | 62,3 |
| | | 2,0 | 25 | 0,326 | 0,062 | 8,9 | 19,5 |
| 9 | I | 0,6 | 54 | 0,183 | 0,026 | 48,9 | 66,2 |
| | | 0,9 | 95 | 0,183 | 0,023 | 48,9 | 70,1 |
| | | 2,0 | 42 | 0,197 | 0,027 | 45,0 | 64,9 |
| Edukt (MDI) | | | | 0,350 | 0,086 | | |

In Vergleichsversuch 1 wird selbst bei relativ geringen Belastungen (0,5 g/ml.h) nur eine geringe Farbaufhellung festgestellt. Als Träger wurden α-Al₂O₃ Kugeln mit einem Durchmesser von 4 bis 5 mm und einer BET-Oberfläche von 10 m²/g (Stand der Technik, EP-A 561 225) eingesetzt. Mit dem gleichen Katalysator wie in Beispiel 1, jedoch auf Durchmesser von 1 bis 2 mm zerkleinert, wird eine erhebliche Farbaufhellung selbst bei hoher Belastung festgestellt (Beispiel 2).

Im Vergleichsbeispiel 3 wird ein Katalysator eingesetzt, der bedingt durch seine Herstellung noch Base (NaOH bzw. Na₂CO₃) enthält. Mit diesem Katalysator wird nur eine mangelhafte Aufhellung erreicht. Außerdem mußte der Versuch nach 65 Stunden wegen Verstopfung des Reaktors abgebrochen werden. Mit einem ähnlichen Katalysator, aber ohne Natriumionen, wird eine große Farbaufhellung erreicht (Beispiel 4).

Ein Katalysator mit zu großen Abmessungen führt nur zu geringen Farbaufhellungen (Vergleichsbeispiel 7) im Gegensatz zu einem Katalysator aus kleinen Kugeln (Beispiel 4).

Ein Katalysator mit einer zu großen BET-Oberfläche liefert ebenfalls mangelhafte Ergebnisse (Vergleichsbeispiel 8) im Gegensatz zu Katalysatoren mit geringer Oberfläche (Beispiele 2, 5 und 6).

Durch die Mitverwendung von Gold als Katalysatorkomponente läßt sich die Aktivität noch steigern (Beispiel 9 im Vergleich zu Beispiel 6).

## Patentansprüche

1. Verfahren zur Herstellung von Isocyanaten oder Isocyanatgemischen, die keine farbgebenden Stoffe enthalten, durch Phosgenierung der entsprechenden Amine oder Amingemische und anschließende Behandlung der so hergestellten Isocyanate oder Isocyanatgemische mit Wasserstoff in Gegenwart von Trägerkatalysatoren, dadurch gekennzeichnet, daß als Trägerkatalysatoren Schalenkatalysatoren eingesetzt werden, die in der kürzesten Ausdehnung 0,3 bis 3 mm groß sind, BET-Oberflächen von 0,5 bis 150 m²/g aufweisen und alkalifrei sind.

2. Verfahren gemäß Anspruch 1, dadurch gekennzeichnet, daß das Isocyanat oder Isocyanatgemisch und der Wasserstoff so mit dem Trägerkatalysator in Berührung gebracht werden, daß die flüssige Phase von oben nach unten durch das Katalysatorbett rieselt, während die gasförmige Phase von unten nach oben oder vorzugsweise von oben nach unten strömt.

## Claims

1. Method for the preparation of isocyanates or isocyanate mixtures which contain no colouring substances by phosgenation of the corresponding amines or amine mixtures and subsequent treatment of the isocyanates or isocyanate mixtures thus prepared with hydrogen in the presence of supported catalysts, characterised in that the supported catalysts used are shell catalysts which are from 0.3 to 3 mm in length along the shortest dimension, have BET surface areas of from 0.5 to 150 m²/g and are alkali-free.

2. Method according to claim 1, characterised in that the isocyanate or isocyanate mixture and the hydrogen are brought into contact with the supported catalyst in such a way that the liquid phase trickles through the catalyst bed from top to bottom, while the gaseous phase flows from bottom to top or preferably from top to bottom.

## Revendications

1. Procédé pour la préparation d'isocyanates ou de mélange d'isocyanates débarrassés de substances colorantes par phosgénation des amines ou mélange d'amines correspondants suivie du traitement des isocyanates ou mélanges d'isocyanates obtenus par l'hydrogène en présence de catalyseurs sur supports, caractérisé en ce que l'on utilise en tant que catalyseurs sur supports des catalyseurs superficiels (en enveloppes) qui, dans l'extension la plus courte, ont une dimension de 0,3 à 3 mm, avec une surface BET de 0,5 à 150 m²/g, et qui sont exempts d'alcalis.

2. Procédé selon revendication 1, caractérisé en ce que l'isocyanate ou mélange d'isocyanates et l'hydrogène sont mis en contact avec le catalyseur sur support par ruissellement de la phase liquide de haut en bas au travers du lit de catalyseur cependant que la phase gazeuse circule de bas en haut ou, de préférence, de haut en bas.
